# EUROPEAN PATENT APPLICATION

(11) **EP 4 759 147 A2**
(43) Date of publication of application: **17.06.2026**
(21) Application number: 26167541.7
(22) Date of filing: 19.11.2021
(51) Int. Cl.: A23L 19/00

(54) **TEXTURE-CONTROLLED FIBER INGREDIENT CONTAINING VISCOUS SOLUBLE FIBER AND CONSUMABLE FOOD AND FIBER SUPPLEMENT PRODUCTS INCORPORATING SAME**

(30) Priority: 20.11.2020 US 202063116401 P; 16.11.2021 US 202163279935 P
(62) Divisional of application: 21827316.7
(71) Applicant: General Mills, Inc., Minneapolis, Minnesota 55426 (US)
(72) Inventor: GALUSKA, Peter J., Hudson, 54016 (US); GOEDEKEN, Douglas, L., Blaine, 55449 (US); GUGGER, Eric, T., Plymouth, 55442 (US); HUBER, Jeffrey, Robbinsdale, 55422 (US); WHITMAN, Scott, New Hope, 55427 (US)
(74) Representative: Dehns

(57) **Abstract**

A texture-controlled fiber ingredient containing a viscous soluble fiber and a non-crosslinked acid-reversible gel-forming compound is described. The ingredient can be contacted with a divalent cation solution and combined with a food ingredient or edible ingredient suitable for use in a supplement to produce a viscous fiber fortified food or fiber supplement. In some versions of a viscous fiber fortified food or fiber supplement, the viscous soluble fiber remains partially hydrated over shelf life of the food, and maintains a desirable texture over the shelf life. Some versions of a food mix or fiber supplement are in dry form.

## Description

### TECHNICAL FIELD

This disclosure generally relates to food ingredients and fiber supplements containing viscous soluble fiber, and foods and fiber supplements that contain viscous soluble fiber while maintaining an acceptable texture.

### BACKGROUND

Viscous soluble fibers are produced by many plants and microorganisms. Seeds known to contain higher quantities of viscous soluble fiber include psyllium, flax, chia, okra, fenugreek, and holy basil, among others. In seeds, viscous soluble fiber acts to absorb and retain moisture to facilitate germination and other functions. Other sources of viscous soluble fibers include, for example, non-seed plant parts, such as konjac root, and microbe-based products, such as bacterial or yeast fermentation products. Viscous soluble fiber can also provide health benefits in human diets, such as cholesterol reduction, blood glucose management, weight management, and digestive health. Viscous soluble fiber supplements are available in the form of gel capsules, powdered drink mixes, cookie biscuits, and snack bars. However, current soluble fiber supplements each have at least one major disadvantage that discourages consumers from using them. A disadvantage of gel capsules is the quantity, often 5 or more capsules, that must be consumed in a day to achieve an effective dose. A disadvantage of current edible formulations is that psyllium and other sources of mucilage absorb moisture rapidly and become viscous. Powdered drink mixes must be consumed immediately following water addition or the product becomes very thick and unpleasant. In cookie biscuits and bars containing psyllium, these products can be difficult to manufacture due to viscosity issues, and also become viscous and gummy in the mouth upon chewing and when coming in contact with saliva, causing sticking to teeth and making consumption unpleasant, and sometimes even creating a choking hazard.

Previous approaches have been described which attempt to manage the moisture absorption of viscous soluble fiber, particularly for psyllium containing products. However, these approaches demonstrate only modest improvement in moisture uptake for certain manufacturing processes, extension of gelation time for powdered drink mixes during mixing, and providing moisture barriers to psyllium hydration when part of high fat formulations. There is still a need for processes and formulations that improve the consumer eating experience and avoiding unpleasant viscosity in the mouth, especially for foods that are not high in fat or in supplements that require chewing or hydration prior to consumption.

### SUMMARY OF THE INVENTION

A texture-controlled fiber ingredient is provided herein. The texture-controlled fiber ingredient is an edible dry composition containing a viscous soluble fiber source and a non-crosslinked acid-reversible gel-forming compound at a ratio of 30:1 to 6:1 by dry weight viscous soluble fiber source to acid-reversible gel-forming compound.

The viscous soluble fiber source can include, for example, psyllium husk, beta-glucan, guar gum, konjac, or hydroxypropyl methylcellulose. The acid-reversible gel-forming compound can include pectin or alginate.

A method of making a viscous soluble fiber fortified food is also provided. The method includes providing a food ingredient, combining the food ingredient with a texture-controlled fiber ingredient, and contacting the texture-controlled fiber ingredient with a divalent cation solution in an amount sufficient to cross-link an acid-reversible gel-forming compound in the texture-controlled fiber ingredient. In some embodiments, the divalent cation solution can be combined with the food ingredient prior to combining the food ingredient with the texture-controlled fiber ingredient. In some embodiments, the divalent cation solution is contacted with the texture-controlled fiber ingredient prior to combining the texture-controlled fiber ingredient with the food ingredient. In some embodiments, the food ingredient can include a fruit ingredient to make a fruit snack, such as a gummy snack. In some embodiments, the divalent cation solution can include Ca2+ or Mg2+.

A viscous soluble fiber fortified food is provided herein. The viscous soluble fiber fortified food includes a viscous soluble fiber system, the viscous soluble fiber system that includes a viscous soluble fiber source in an amount of at least 2% by dry weight of the food, where the viscous soluble fiber source is partially hydrated, and an acid-reversible gel encapsulating the viscous soluble fiber source, the acid-reversible gel being in an amount to provide a ratio of 30:1 to 6:1 by dry weight viscous soluble fiber source to acid-reversible gel, where the ion-reversible gel maintains the viscous soluble fiber source in a partially hydrated state over a shelf life of at least 30 days. In some embodiments, the viscous soluble fiber fortified food can have a moisture content of 10-30%. In some embodiments, the viscous soluble fiber source can be included in an amount of from about 5% to about 30% by weight of the food. In some embodiments, the viscous soluble fiber source can include psyllium husk, beta-glucan, guar gum, konjac, or hydroxypropyl methylcellulose. In some embodiments, the acid-reversible gel can include crosslinked pectin or crosslinked alginate. In some embodiments, the food can be a fruit snack, such as a gummy snack. In some embodiments, the fruit snack can contain from about 2% to about 10% by weight viscous soluble fiber source.

A method of making a texture-controlled fiber ingredient is also provided. The method includes producing a homogenous composition that contains an aqueous plasticizer, a viscous soluble fiber source, and a non-crosslinked acid-reversible gel-forming compound, where the viscous soluble fiber source and non-crosslinked acid-reversible gel-forming compound are included at a ratio of 30:1 to 6:1 by dry weight viscous soluble fiber source to acid-reversible gel-forming compound; and drying the composition to a moisture content of less than 12% to produce the texture-controlled fiber ingredient. In some embodiments, part or all of the step of producing a homogenous composition can be performed at a temperature above 170° F (e.g., about 180° F to about 280° F). In some embodiments the homogenous composition can have a moisture content of about 40% to about 90% (e.g., about 50% to about 75%). In some embodiments, the non-crosslinked acid-reversible gel-forming compound can be dissolved in the aqueous plasticizer to form a solution, and the solution is combined with the viscous soluble fiber source. In some embodiments, the composition can be formed into pieces prior to drying. In some embodiments, the homogenous composition can be produced in an extruder. In some embodiments, the texture-controlled fiber ingredient can be ground into a powder. In some embodiments, the composition can be dried by spray drying.

A fiber supplement is also provided herein. A fiber supplement can contain a viscous soluble fiber system, the viscous soluble fiber system consisting of a partially hydrated viscous soluble fiber source and an acid-reversible gel encapsulating the partially hydrated viscous soluble fiber source, the acid-reversible gel included at a ratio of 30:1 to 6:1 by dry weight viscous soluble fiber source to acid-reversible gel, and maintaining the viscous soluble fiber source in a partially hydrated state over a shelf life of at least 30 days. A fiber supplement can contain a dehydrated viscous soluble fiber system, wherein the dehydrated viscous soluble fiber system comprises a viscous soluble fiber source and a non-crosslinked acid-reversible gel-forming compound at a ratio of 30:1 to 6:1 by dry weight viscous soluble fiber source to acid-reversible gel-forming compound. A fiber supplement can contain a texture-controlled fiber ingredient and a divalent cation, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound.

A viscous soluble fiber source in a fiber supplement provided herein can include psyllium husk, beta-glucan, guar gum, konjac, or hydroxypropyl methylcellulose, and the acid-reversible gel-forming compound comprises pectin or alginate. In some embodiments, a viscous soluble fiber source can include psyllium husk and the acid-reversible gel or acid-reversible gel-forming compound can include pectin.

In some embodiments, a fiber supplement can have a moisture content of 10% or less. In some embodiments, a fiber supplement can be formulated to be hydrated prior to or during consumption. In some embodiments, a fiber supplement can be a beverage mix, a chewable tablet, or a lozenge.

In some embodiments, a fiber supplement can have a moisture content of at least 10%. In some embodiments, a fiber supplement can be a jelly or gummy supplement, a beverage, or a snack bar.

In some embodiments of a fiber supplement, at least a portion of a texture-controlled fiber ingredient can be included in a coating.

In some embodiments, a fiber supplement can include at least 1 g, or at least 1.7 g psyllium soluble fiber per serving.

A method of relieving constipation in a patient is also provided herein. The method includes administering at least one serving of a fiber supplement or a viscous soluble fiber fortified food provided herein at least once a day to a patient suffering from constipation until constipation is relieved.

A method of reducing blood cholesterol levels in a patient is provided herein. The method includes administering at least one serving of a fiber supplement or a viscous soluble fiber fortified food provided herein at least once a day to a patient suffering high blood cholesterol levels until blood cholesterol levels in the patient are reduced.

A method of increasing satiety in a patient is provided herein. The method includes administering at least one serving of a fiber supplement or a viscous soluble fiber fortified food provided herein at least once a day to the patient prior to at least one meal a day, the fiber supplement containing an effective amount of psyllium husk to increase satiety.

A fiber supplement for use in relieving constipation in a patient is provided herein. The fiber supplement includes at least 1 g, or at least 1.7 g psyllium soluble fiber per serving, and includes: a viscous soluble fiber system, the viscous soluble fiber system consisting of a partially hydrated psyllium husk and an acid-reversible gel encapsulating the partially hydrated psyllium husk, the acid-reversible gel included at a ratio of 30:1 to 6:1 by dry weight psyllium husk to acid-reversible gel, and maintaining the psyllium husk in a partially hydrated state over a shelf life of at least 30 days;
a dehydrated viscous soluble fiber system, wherein the dehydrated viscous soluble fiber system comprises psyllium husk and a non-crosslinked acid-reversible gel-forming compound at a ratio of 30:1 to 6:1 by dry weight psyllium husk to acid-reversible gel-forming compound; or
a texture-controlled fiber ingredient and a divalent cation, the texture-controlled fiber ingredient being an edible dry composition consisting essentially of psyllium husk and a non-crosslinked acid-reversible gel-forming compound at a ratio of 30:1 to 6:1 by dry weight viscous soluble fiber source to acid-reversible gel-forming compound.

A fiber supplement for use in reducing blood cholesterol levels in a patient is provided herein. The fiber supplement includes at least 1 g, or at least 1.7 g psyllium soluble fiber per serving, and includes: a viscous soluble fiber system, the viscous soluble fiber system consisting of a partially hydrated psyllium husk and an acid-reversible gel encapsulating the partially hydrated psyllium husk, the acid-reversible gel included at a ratio of 30:1 to 6:1 by dry weight psyllium husk to acid-reversible gel, and maintaining the psyllium husk in a partially hydrated state over a shelf life of at least 30 days;
a dehydrated viscous soluble fiber system, wherein the dehydrated viscous soluble fiber system comprises psyllium husk and a non-crosslinked acid-reversible gel-forming compound at a ratio of 30:1 to 6:1 by dry weight psyllium husk to acid-reversible gel-forming compound; or
a texture-controlled fiber ingredient and a divalent cation, the texture-controlled fiber ingredient being an edible dry composition consisting essentially of psyllium husk and a non-crosslinked acid-reversible gel-forming compound at a ratio of 30:1 to 6:1 by dry weight viscous soluble fiber source to acid-reversible gel-forming compound.

A fiber supplement for use in increasing satiety in a patient is provided herein. The fiber supplement includes at least 6 g, or at least 7 g psyllium husk per serving, and includes:
a viscous soluble fiber system, the viscous soluble fiber system consisting of a partially hydrated psyllium husk and an acid-reversible gel encapsulating the partially hydrated psyllium husk, the acid-reversible gel included at a ratio of 30:1 to 6:1 by dry weight psyllium husk to acid-reversible gel, and maintaining the psyllium husk in a partially hydrated state over a shelf life of at least 30 days;
a dehydrated viscous soluble fiber system, wherein the dehydrated viscous soluble fiber system comprises psyllium husk and a non-crosslinked acid-reversible gel-forming compound at a ratio of 30:1 to 6:1 by dry weight psyllium husk to acid-reversible gel-forming compound; or
a texture-controlled fiber ingredient and a divalent cation, the texture-controlled fiber ingredient being an edible dry composition consisting essentially of psyllium husk and a non-crosslinked acid-reversible gel-forming compound at a ratio of 30:1 to 6:1 by dry weight viscous soluble fiber source to acid-reversible gel-forming compound.

In some embodiments, any of the fiber supplements provided herein can have a viscous soluble fiber source included in an amount of from about 2% to about 30% or about 3% to about 10% by dry weight of the fiber supplement.

A method of making a fiber supplement is also provided herein. The method includes:
combining an edible ingredient with a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound; contacting the texture-controlled fiber ingredient with a divalent cation solution in an amount sufficient to cross-link the acid-reversible gel-forming compound to form a first composition comprising the edible ingredient and a viscous soluble fiber system, the viscous soluble fiber system consisting of a partially hydrated viscous soluble fiber source and an acid-reversible gel encapsulating the partially hydrated viscous soluble fiber source; and forming the first composition into the fiber supplement;
combining the edible ingredient with a dehydrated viscous soluble fiber system to form a second composition, wherein the dehydrated viscous soluble fiber system comprises psyllium husk and a non-crosslinked acid-reversible gel-forming compound at a ratio of 30:1 to 6:1 by dry weight psyllium husk to acid-reversible gel-forming compound; or
combining the edible ingredient with the texture-controlled fiber ingredient and a divalent cation source to form a third composition; and forming the third composition into the fiber supplement.

In some embodiments of a method of making a supplement, the divalent cation solution can be combined with the edible ingredient prior to combining the edible ingredient with the texture-controlled fiber ingredient. In some embodiments, the divalent cation solution can be contacted with the texture-controlled fiber ingredient prior to combining the texture-controlled fiber ingredient with the edible ingredient.

In some embodiments of a method of making a supplement, the divalent cation solution can include Ca2+ or Mg2+.

In some embodiments of any of the methods of making a supplement described herein, the edible ingredient can include a fruit ingredient to make a fruit-based fiber supplement. In some embodiments, a fruit-based fiber supplement can be a gummy fiber supplement.

A method of making a dehydrated viscous soluble fiber system is provided herein. The method includes providing a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound; contacting the texture-controlled fiber ingredient with a divalent cation solution in an amount sufficient to cross-link the acid-reversible gel-forming compound to form a composition, wherein contact of the divalent cation solution with the texture-controlled fiber ingredient is insufficient to cross-link at least a portion of the acid-reversible gel-forming compound; and drying the viscous soluble fiber system to a moisture content of less than 12% to form the dehydrated viscous soluble fiber system, wherein the dehydrated viscous soluble fiber system includes the viscous soluble fiber source and at least a portion of the of the acid-reversible gel-forming compound that is non-crosslinked.

A method of making a viscous soluble fiber fortified food is provided herein. The method includes providing a food ingredient, combining the food ingredient with a dehydrated viscous soluble fiber system, the dehydrated viscous soluble fiber system being an edible dry composition consisting essentially of a viscous soluble fiber source, acid-reversible gel-forming compound, and a divalent cation in an amount sufficient to cross-link the acid-reversible gel-forming compound, the ratio of viscous soluble fiber source to acid-reversible gel-forming compound being 30:1 to 6:1 by dry weight, where at least a portion of the of the acid-reversible gel-forming compound is non-crosslinked; contacting the dehydrated viscous soluble fiber system with water in an amount sufficient to produce a viscous soluble fiber system in a composition, where the viscous soluble fiber system consists of the viscous soluble fiber source in an amount of at least 2% by dry weight of the food, the viscous soluble fiber source being partially hydrated; and an acid-reversible gel encapsulating the viscous soluble fiber source, the acid-reversible gel maintaining the viscous soluble fiber source in a partially hydrated state over a shelf life of at least 30 days; and producing a food from the composition. In some embodiments, the water can be combined with the food ingredient prior to combining the food ingredient with the dehydrated viscous soluble fiber system. In some embodiments, the water can be contacted with the dehydrated viscous soluble fiber system prior to combining the dehydrated viscous soluble fiber system with the food ingredient. In some embodiments, the viscous soluble fiber source can be included in an amount of from about 5% to about 30% by weight of the food. In some embodiments, the viscous soluble fiber source can include psyllium husk. In some embodiments, the acid-reversible gel can include crosslinked pectin. In some embodiments, the viscous soluble fiber source can contain beta-glucan, guar gum, konjac, or hydroxypropyl methylcellulose. In some embodiments, the acid-reversible gel can include crosslinked alginate.

Also provided herein is a mix, kit, or product suitable for making a viscous soluble fiber fortified food. A mix, kit, or product suitable for making a viscous soluble fiber fortified food contains at least one food ingredient, and:
a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound; or
dehydrated viscous soluble fiber system, the dehydrated viscous soluble fiber system being an edible dry composition consisting essentially of a viscous soluble fiber source, acid-reversible gel-forming compound, and a divalent cation in an amount sufficient to cross-link the acid-reversible gel-forming compound, the ratio of viscous soluble fiber source to acid-reversible gel-forming compound being 30:1 to 6:1 by dry weight, where at least a portion of the of the acid-reversible gel-forming compound is non-crosslinked.

In some embodiments, a mix, kit, or product suitable for making a viscous soluble fiber fortified food can contain a texture-controlled fiber ingredient and a divalent cation.

### DETAILED DESCRIPTION

Foods containing viscous soluble fibers (also referred to mucilaginous fibers) in amounts sufficient to provide a single serving fiber benefit (e.g., 1 gram to 10 grams, or 1.5 grams to 7 grams per serving) suffer from an unpleasant texture, often described as slimy, sticky, or viscous, due to water absorption by the viscous soluble fiber in the food itself and/or upon contact with saliva during eating. Previous attempts at improving eating qualities of viscous soluble fibers were directed to using complicated combinations of different fibers, and optionally fillers and/or sweeteners, and spray drying methods to result in multilayered particulates that do not swell upon addition to moist foods. However, not only are such fiber particulates complicated to make, they would be conspicuous in foods with a smooth texture, such as gummy snacks and fruit bites. In addition, because such particulates do not swell at all in a moist environment, they may provide a grainy texture in baked goods, bars, and other foods when the particulates are added at levels sufficient to provide a single serving fiber benefit.

Fiber supplements containing viscous soluble fibers can suffer from similar problems as foods that contain viscous soluble fibers. Fiber supplements containing viscous soluble fibers, such as psyllium soluble fiber, often need to be consumed quickly with a large quantity of water to ensure that the fiber doesn't swell so much that the consumer ends up eating or drinking a slimy-textured concoction or have difficulties swallowing the supplement. Supplements that have higher moisture content, such as gummy-type supplements, or supplements that are meant to be chewed, avoid the use of viscous soluble fibers due to these issues and instead rely on lower viscosity ingredients, such as inulin, soluble corn fiber, to achieve related, but not identical benefits compared to viscous soluble fibers.

It has surprisingly been discovered, and is disclosed herein, that by combining a viscous soluble fiber (e.g., psyllium, guar gum, hydroxypropyl methylcellulose, konjac, or beta-glucan) with an acid-reversible gel-forming compound (e.g., low methoxy pectin, low ester pectin, or alginate) that is not crosslinked, a fiber ingredient can be made that reduces, but does not fully prevent, moisture absorption and swelling of the viscous soluble fiber in the presence of water and a divalent cation (e.g., Ca²⁺ or Mg²⁺). As a result, the fiber ingredient is texture-controlled. That is, such a texture-controlled fiber ingredient can be included in foods that contain water without imparting a slimy or viscous texture, and without a grainy or conspicuous texture. In some cases, a texture-controlled fiber ingredient or dehydrated viscous soluble fiber system described herein can be included in a food, where the texture-controlled fiber ingredient or dehydrated viscous soluble fiber system provides a texture that resembles hydrated tapioca pearls without imparting a slimy or viscous texture, and without imparting a grainy texture.

In addition, it is particularly surprising that a texture-controlled ingredient provided herein can enable production of a gummy-type product (e.g., a gummy snack or a gummy supplement), since including a viscous soluble fiber had otherwise made it too difficult to manufacture such products. That is, a viscous soluble fiber such as psyllium would ordinarily increase viscosity during manufacture that causes problems with pumpability, deposit into molds, and the like. A texture-controlled ingredient provided herein reduces viscosity during manufacture to provide the ability to include a significant amount of viscous soluble fiber, such as psyllium, in a gummy-type product without hindering manufacturability.

Without being bound by theory, it is believed that when a viscous soluble fiber and an acid-reversible gel-forming compound are combined to interact in the presence of water and in the absence of a divalent cation to form a composition, and then the composition is dried, a resulting texture-controlled fiber ingredient allows for the viscous soluble fiber to partially hydrate, but not fully hydrate, when exposed to water and a divalent cation. A texture-controlled fiber ingredient may benefit when the viscous soluble fiber and the acid-reversible gel-forming compound are combined at temperatures exceeding 170° F (e.g., 175° F- 300° F, or 180° F - 280° F). As a result, the unpleasant texture (e.g., slimy or viscous texture) of a fully hydrated viscous soluble fiber can be avoided while preventing a conspicuous or gritty texture that can be present when the viscous soluble fiber is prevented from hydrating or swelling at all.

Importantly, although a viscous soluble fiber in a texture-controlled fiber ingredient can remain partially hydrated in the presence of water and a divalent cation for at least several hours (e.g., several days, or several months), which can enable an extended shelf life in a food or supplement without significantly affecting texture, the viscous soluble fiber can fully hydrate upon exposure to the acid environment of the stomach as the acid-reversible gel-forming compound is degraded. As a result, a viscous soluble fiber in a texture-controlled fiber ingredient can be made available in the gastrointestinal tract to provide beneficial functions, such as relief from constipation, cholesterol reduction, and the like.

A texture-controlled fiber ingredient provided herein includes a viscous soluble fiber source and a non-crosslinked acid-reversible gel-forming compound at a ratio of 30:1 to 6:1 (e.g., 25:1 to 8:1, or about 20:1) viscous soluble fiber source to non-crosslinked acid-reversible gel-forming compound by dry weight of the texture-controlled fiber ingredient. In some embodiments, a texture-controlled fiber ingredient can consist essentially of a viscous soluble fiber source and a non-crosslinked acid-reversible gel-forming compound. As used herein, a texture-controlled fiber ingredient is understood to consist essentially of a viscous soluble fiber source and a non-crosslinked acid-reversible gel-forming compound if it contains at least 92% (e.g., at least 95%, or at least 98%) of a combination of the viscous soluble fiber source and the non-crosslinked acid-reversible gel-forming compound by dry weight of the texture-controlled fiber ingredient. A texture-controlled fiber ingredient consisting essentially of a viscous soluble fiber source and a non-crosslinked acid-reversible gel-forming compound can contain up to 10% (e.g., up to 8%) non-functional compounds (e.g., sugar, starch, flavorants, colorants, and the like) by dry weight of the texture-controlled fiber ingredient. As used herein, non-functional compounds are compounds in a texture-controlled fiber ingredient that do not function to cross-link an included acid-reversible gel-forming compound. For example, a texture-controlled fiber ingredient can include up to 8% by dry weight sugar or starch that is often included as a filler ingredient in an acid-reversible gel-forming compound, such as pectin. In another example, a flavorant, such as cocoa powder or cinnamon, can be added to provide flavor without affecting the function of a texture-controlled fiber ingredient.

A texture-controlled fiber ingredient provided herein can be in a dry form having a moisture content of 12% or less (e.g., 10% or less, about 2% to about 10%, or about 8% to about 9%) by weight. In some embodiments, a texture-controlled fiber ingredient can be a powder, in sheet form, or in pellet form. Preferably, a texture-controlled fiber ingredient that is not to be used immediately in a food can be packaged in moisture-resistant packaging to prevent atmospheric moisture from being absorbed by the texture-controlled fiber ingredient.

As used herein, a viscous soluble fiber source can include whole ground viscous soluble fiber-containing seeds (e.g., psyllium seeds, chia seeds, flax seeds, and the like, or combinations thereof), viscous soluble fiber-containing seed husks (e.g., psyllium husk, chia husk, flax husk, and the like, or combinations thereof), viscous soluble fiber-containing roots (e.g., konjac root, and the like), or fiber concentrates or isolates (e.g., psyllium soluble fiber, beta-glucan, guar gum, konjac mannan, or hydroxypropyl methylcellulose). However, in more preferred embodiments, a viscous soluble fiber source comprises more concentrated soluble viscous fiber sources, such as ground seed husks, fiber concentrates, or fiber isolates. Psyllium husk that is at least 85% pure (e.g., at least 90% pure or at least 95% pure) is particularly suitable. Commercially available psyllium includes, for example, French psyllium (also referred to as black psyllium; *Plantago indica),* Spanish psyllium (*P. psyllium),* and Indian psyllium (also referred to as blond psyllium; *P. ovata*)*.* The viscous soluble fiber content of whole psyllium seed varies: French psyllium, 11.8%; Indian psyllium, 30.9%; and German psyllium, 11.5%. Psyllium seed husk that is 95% pure is typically around about 60% to about 70% psyllium soluble fiber.

In some embodiments, a viscous soluble fiber source can have a particle size in which more than 90% (e.g., 95% or 99%) of the particles pass through a 40 mesh screen, or more than 90% (e.g., 95% or 99%) of the particles pass through an 80 mesh screen.

As used herein, an acid-reversible gel-forming compound refers to a compound, such as pectin (e.g., low methoxy pectin or low ester pectin) or alginate, that is crosslinked to form a gel upon exposure to water and a divalent cation, where the gel degrades in the presence of a concentrated gastric acid environment (e.g., the stomach). A non-crosslinked acid-reversible gel-forming compound refers to an acid-reversible gel-forming compound that is not crosslinked but is capable of being crosslinked in the presence of water and a divalent cation.

A texture-controlled fiber ingredient can be used to make a viscous soluble fiber fortified food or fiber supplement. A texture-controlled fiber ingredient can be used to make a viscous soluble fiber fortified food by combining at least one food ingredient with the texture-controlled fiber ingredient and contacting the texture-controlled fiber ingredient with a divalent cation solution in an amount sufficient to crosslink an acid-reversible gel-forming compound in the texture-controlled fiber ingredient to make a composition that can be formed, cooked, or otherwise prepared into a food. Such a food can benefit from being able to have a relatively high moisture content (e.g., at least 10% moisture content), and include a viscous soluble fiber system that includes a partially hydrated viscous soluble fiber source over an extended shelf life. Generally, a viscous soluble fiber fortified food should be formulated to avoid interfering with development of an acid-reversible gel and/or avoid degradation of an acid-reversible gel. For example, a viscous soluble fiber fortified food preferably has a pH of at least 4. Alternatively, a low pH food, such as a yogurt or carbonated drink can include excess soluble calcium to ensure development and maintenance of an acid-reversible gel. In some embodiments, high acid and/or high salt ingredients can be placed on the outside of a food viscous soluble fiber fortified food (e.g., "sanded" with citric acid or malic acid powder) to provide a desired flavor (e.g., sour) or visual impact without interfering with development of an acid-reversible gel and/or causing degradation of an acid-reversible gel.

In some embodiments, a divalent cation solution can be combined with a food ingredient prior to combining the food ingredient with a texture-controlled food ingredient. In some embodiments, a texture-controlled food ingredient can be contacted with a divalent cation solution prior to being combined with a food ingredient.

A texture-controlled fiber ingredient can be used to make a fiber supplement by combining at least one edible ingredient suitable for inclusion in a dietary supplement (e.g., a binder, a plasticizer, a food ingredient, or the like) with the texture-controlled fiber ingredient and contacting the texture-controlled fiber ingredient with a divalent cation solution in an amount sufficient to crosslink an acid-reversible gel-forming compound in the texture-controlled fiber ingredient to make a composition that can be formed, cooked, or otherwise prepared into a fiber supplement. A fiber supplement made according to this method can benefit from being able to have a relatively high moisture content (e.g., at least 10% moisture content), and include a viscous soluble fiber system that includes a partially hydrated viscous soluble fiber source over an extended shelf life. As with a viscous soluble fiber fortified food, a fiber supplement should be formulated to avoid interfering with development of an acid-reversible gel and/or avoid degradation of an acid-reversible gel.

Alternatively, a controlled fiber ingredient can be used to make a fiber supplement by combining at least one edible ingredient with a dehydrated viscous soluble fiber system to produce a composition that can be formed, cooked, or otherwise prepared into a fiber supplement. A fiber supplement made according to this method can benefit from having a low moisture content, yet allow partial hydration of an included viscous soluble fiber source upon exposure to moisture (e.g., upon chewing or mixing with a liquid), and crosslinking of an included acid-reversible gel-forming compound as divalent cations interact with the acid-reversible gel-forming compound to prevent full hydration of the viscous soluble fiber source, such that the supplement provides a pleasant, non-slimy or overly viscous texture.

As used herein, a dehydrated viscous soluble fiber system refers to dry composition made from a texture-controlled fiber ingredient that has been contacted with a divalent cation solution in an amount sufficient to cross-link the acid-reversible gel-forming compound and then dried without allowing all of the acid-reversible gel-forming compound to be crosslinked. A dehydrated viscous soluble fiber system can include up to 10% (e.g., up to 8%) non-functional compounds, similarly to a texture-controlled fiber ingredient. This can be achieved using any appropriate method, such spraying pellets of texture-controlled fiber ingredient with divalent cation solution and then drying so that only the surface of the pellets are exposed to divalent cation solution, leaving any acid-reversible gel-forming compound non-crosslinked. The pellets may then be used as-is, or ground to a powder for use.

That is, a dehydrated viscous soluble fiber system includes a viscous soluble fiber source, a non-crosslinked acid-reversible gel-forming compound, and divalent cation. In some cases, a portion of the acid-reversible gel-forming compound may be crosslinked to form a gel prior to drying, but does not fully encapsulate an included viscous soluble fiber source. Such a dehydrated viscous soluble fiber system may provide a benefit of enabling a dry supplement format while ensuring that sufficient acid-reversible gel-forming compound is crosslinked during consumer use (e.g., mixing with a liquid or chewing) that the included viscous soluble fiber source does not fully hydrate.

Without being bound by theory, it is believed that a dehydrated viscous soluble fiber system described herein may function similarly to a texture-controlled fiber ingredient in that, although it contains a texture-controlled fiber ingredient that has been contacted with a divalent cation solution in an amount that is sufficient to cause crosslinking of an included acid-reversible gel-forming compound, followed by drying, at least some of the acid-reversible gel-forming compound remains non-crosslinked, which allows the viscous soluble fiber to partially, but not fully, hydrate when later exposed to water (e.g., water, high moisture ingredients, or saliva). Advantageously, a dehydrated viscous soluble fiber system can, in some embodiments, be included as an ingredient in a food. As an ingredient in a food a dehydrated viscous soluble fiber system can be rehydrated and combined with other food ingredients to produce food that includes a viscous soluble fiber system and enjoy the same benefits as a food that has included a texture-controlled fiber ingredient.

In another alternative, a controlled fiber ingredient can be used to make a fiber supplement by combining at least one edible ingredient with a texture-controlled fiber ingredient and a divalent cation source to produce a composition that can be formed, cooked, or otherwise prepared into a fiber supplement. A fiber supplement made according to this method can benefit from having a low moisture content, yet allow partial hydration of an included viscous soluble fiber source upon exposure to moisture (e.g., upon chewing or mixing with a liquid), and crosslinking of an included acid-reversible gel-forming compound as divalent cations interact with the acid-reversible gel-forming compound to prevent full hydration of the viscous soluble fiber source, such that the supplement provides a pleasant, non-slimy or overly viscous texture.

A viscous soluble fiber fortified food or fiber supplement made with a texture-controlled fiber ingredient can contain at least 2% (e.g., about 5% to about 30%, about 5% to about 25%, or about 5% to about 10%) viscous soluble fiber source by weight of the food. In some embodiments, a viscous soluble fiber fortified food can contain sufficient soluble fiber source per serving to provide a health benefit, such as relief from constipation or cholesterol reduction, when at least a single serving of the viscous soluble fiber fortified food is consumed daily. For example, a viscous soluble fiber fortified food can contain at least 1 g (e.g., at least 1.7 g) psyllium soluble fiber (e.g., at least 2.4 g psyllium husk) per serving for consumption of at least 1 serving (e.g., 1 to 4 servings) per day to achieve relief from constipation and/or cholesterol reduction, or at least 6 g (e.g., at least 7 g) psyllium husk per serving to achieve appetite suppression and/or satiety. In another example, a viscous soluble fiber fortified food can be formulated to achieve a health benefit in a child. For example, a viscous soluble fiber fortified food can contain at least 1 g psyllium soluble fiber (e.g., at least 1.4 g psyllium husk) per serving for consumption of at least 1 serving (e.g., 1 to 4 servings) per day to achieve relief from constipation in a child.

Some embodiments of a viscous soluble fiber fortified food or a fiber supplement made with a texture-controlled fiber ingredient includes a viscous soluble fiber system that consists of a viscous soluble fiber source that is partially hydrated, and an acid-reversible gel (i.e., an acid-reversible gel-forming compound that is crosslinked in the presence of water and a divalent cation) that at least partially encapsulates the viscous soluble fiber source and maintains the viscous soluble fiber source in a partially hydrated state over the shelf life of the food. In some embodiments, a viscous soluble fiber fortified food or a fiber supplement that includes a viscous soluble fiber system can have a shelf life of at least 15 days (e.g., at least 30 days, or at least 90 days). Beneficially, because a viscous soluble fiber source can be maintained in a partially hydrated state, a viscous soluble fiber fortified food or fiber supplement can maintain a desirable texture over the shelf life of the food or supplement despite having a relatively high moisture content (e.g., 10% to 30% by weight).

As used herein, a viscous soluble fiber source is considered partially hydrated when it has absorbed sufficient water to cause swelling of the viscous soluble fiber source, but has not fully hydrated. A viscous soluble fiber source can be observed to be partially hydrated in a viscous soluble fiber fortified food or a fiber supplement if the viscous soluble fiber source has swelled (e.g., as visualized using a microscope), but the viscous soluble fiber fortified food or fiber supplement does not exhibit a slimy viscous texture associated with a viscous soluble fiber. In some cases, the texture of a viscous soluble fiber fortified food or fiber supplement made using an amount of viscous soluble fiber source in a texture-controlled fiber ingredient can be less slimy or viscous compared to a control food or fiber supplement made using the same amount of viscous soluble fiber that is not provided as a texture-controlled fiber ingredient.

A viscous soluble fiber source can be observed to be partially hydrated in a texture-controlled fiber ingredient when the texture-controlled fiber ingredient is contacted with a divalent cation solution to form an acid-reversible gel and swelling is observed (to indicate partial hydration), and upon sufficient acidification to degrade the acid-reversible gel, the viscous soluble fiber source is observed to swell further, indicating complete hydration. It is to be understood that different viscous soluble fibers have different capacities to absorb water and so the water content of a partially hydrated viscous soluble fiber source may differ depending on the fiber source used. For example, depending on the psyllium source, fully hydrated psyllium can result in a swelling capacity of 42-60 ml per gram psyllium, as measured using standard procedure. In some embodiments, a texture-controlled fiber ingredient including pectin and psyllium described herein has been observed to reach a swelling capacity of about 15 ml to about 30 ml per gram of psyllium included in the texture-controlled ingredient when placed in an excess volume of a 0.2% calcium chloride solution. The volume of such a texture-controlled fiber ingredient is less than fully hydrated psyllium, indicating partial hydration, with further hydration (as observed by additional swelling) achievable upon acidification.

A viscous soluble fiber fortified food can be any food, including without limitation, fruit snacks (e.g., gummy snack, fruit bites, and the like), baked goods (e.g., cakes, brownies, cookies, and the like), snack bars (e.g., granola bars, chewy bars, and the like), yogurt, and drinks. Particularly useful are foods that are relatively high in moisture (e.g., about 10% to about 30%), and foods that would exhibit conspicuous changes in appearance or texture if an ingredient that included a viscous soluble fiber source that was prevented from swelling was included. In some cases, a viscous soluble fiber fortified food can include a texture-controlled fiber ingredient in a coating (e.g., compound coating, carbohydrate-based coating, or the like) or filling.

In some embodiments, a texture-controlled fiber ingredient can be combined with one or more food ingredients into a mix, kit, or product that enables a consumer to produce their own viscous soluble fiber fortified food. For example, a texture-controlled fiber ingredient can be combined with ingredient such as flour, sugar, and a divalent cation salt to produce a baked good (e.g., cake or brownie) mix. In another example, a texture-controlled fiber ingredient can be combined with a flavorant, a divalent salt, and a sweetener to produce an instant beverage mix, capsule, or tablet. In another example, a texture-controlled fiber ingredient (e.g., in pellet form) can be combined with a divalent salt with instructions to combine with water to produce a composition resembling tapioca pearls that can be added to a food, such as a drinkable or spoonable yogurt or a tea to make a food that resembles a bubble tea.

In some embodiments, a dehydrated viscous soluble fiber system can be combined with one or more food ingredients into a mix, kit, or product that enables a consumer to produce their own viscous soluble fiber fortified food. A dehydrated viscous soluble fiber system can be used in any way that a texture-controlled fiber ingredient can be used, except that additional divalent cation may not be needed to ensure that a resulting viscous soluble fiber fortified food includes a viscous soluble fiber system without imparting a slimy or viscous texture, and without a grainy or conspicuous texture. In another example, a dehydrated viscous soluble fiber system (e.g., in pellet form) can be combined with water to produce a viscous soluble fiber system to produce a product resembling tapioca pearls. Such a product may be packaged for eating alone or to be added to a food.

A fiber supplement provided herein can be in any form. For example, a fiber supplement can be a dry form (e.g., 10% moisture or less) that is formulated to be hydrated prior to or during consumption. For example, a fiber supplement can be powder that can be added to moist food (e.g., added to a smoothie or shake, sprinkled on a meal, or the like), a beverage mix (e.g.,effervescent powders or tablets, tea-flavored or fruit-flavored mixes, or the like), or a chewable tablet or lozenge. In some embodiments, a fiber supplement can be in a high moisture form (e.g., a moisture content of at least 10%, a moisture content of 10% to more than 90%, or a moisture content of about 10% to about 30%), such as a jelly or gummy supplement (e.g., pectin-based, gelatin-based, starch-based gummy shape, jelly bean, or the like), a beverage (e.g., a fiber-supplemented water or flavored drink, or a smoothie), or a supplement that resembles a food (e.g., a snack bar, or cake bites). In some embodiments, a fiber supplement can include a texture-controlled fiber ingredient in a coating (e.g., compound coating, carbohydrate-based coating, or the like) or filling.

It is to be understood that a viscous soluble fiber fortified food or fiber supplement can contain fiber (e.g., insoluble fiber, soluble fiber) other than a viscous soluble fiber source. Generally, such fibers would not exhibit mucilaginous characteristics.

A texture-controlled fiber ingredient can be made by producing a homogenous composition consisting essentially of an aqueous plasticizer (e.g., water, fruit or vegetable juice, or the like), a viscous soluble fiber source, and a non-crosslinked acid-reversible gel-forming compound, such that the viscous soluble fiber source and the non-crosslinked acid-reversible gel-forming compound are included at a ratio as described in a texture-controlled fiber ingredient above. The composition is then dried to a moisture content of 12% or less to form the texture-controlled fiber ingredient. A texture-controlled fiber ingredient can be optionally formed into an appropriate form (e.g., pellet form, sheeted, or the like) and/or ground into a powder.

In some embodiments, a method of making a texture-controlled fiber ingredient can benefit from producing a homogenous composition at a temperature above 170° F (e.g., about 175° F to about 300° F, about 180° F to about 280° F, or about 190° F to about 260° F) during at least part of the production. Without being bound by theory, it is believed that a temperature above 170° F may improve interaction of a viscous soluble fiber source with a non-crosslinked acid-reversible gel-forming compound such that an acid-reversible gel formed upon contact with water and a divalent cation is more effective at allowing partial, but not full, hydration of the viscous soluble fiber source.

A homogenous composition can have a moisture content of about 40% to about 90%, depending on the desired method of making a texture-controlled fiber ingredient. For example, it was discovered that a texture-controlled fiber ingredient can readily be made in an extruder (e.g., a single screw or a twin screw extruder) by combining a non-crosslinked acid-reversible gel-forming compound in solution to a dry viscous soluble fiber source to make a homogenous composition having a moisture content of about 50% to about 75%, which can then be formed (e.g., sheeted, pellets, ropes, or the like) and dried. In another example, a homogenous composition having a higher moisture content can be dried into a thin film or spray dried to produce a texture-controlled fiber ingredient.

In a further example, psyllium is incorporated into an alginate solution, extruded into a calcium bath to cause alginate gelation, then dried and ground. In another example, psyllium is incorporated into a low ester pectin solution, then dried and ground. In a further example, psyllium is incorporated into a low ester pectin solution, extruded into a calcium bath to cause pectin gelation, then dried and ground.

Thus, this disclosure contains at least the following aspects:
In aspect 1, a texture-controlled fiber ingredient is provided where the texture-controlled fiber ingredient is an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound.

In aspect 2, some embodiments of aspect 1 can include psyllium husk in the viscous soluble fiber source.

In aspect 3, some embodiments of aspect 1 or 2 can include pectin in the acid-reversible gel-forming compound.

In aspect 4, some embodiments of any of aspects 1-3 can include beta-glucan, guar gum, konjac, or hydroxypropyl methylcellulose in the viscous soluble fiber source.

In aspect 5, some embodiments of any of aspects 1-4 can include alginate in the acid-reversible gel-forming compound.

In aspect 6, a method of making a viscous soluble fiber fortified food is provided where the method includes:
a. providing a food ingredient,
b. combining the food ingredient with a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound, and
c. contacting the texture-controlled fiber ingredient with a divalent cation solution in an amount sufficient to cross-link the acid-reversible gel-forming compound.

In aspect 7, some embodiments of aspect 6 include combining the divalent cation solution with the food ingredient prior to combining the food ingredient with the texture-controlled fiber ingredient.

In aspect 8, some embodiments of aspect 6 include contacting the divalent cation solution with the texture-controlled fiber ingredient prior to combining the texture-controlled fiber ingredient with the food ingredient.

In aspect 9, some embodiments of any of aspects 6-8 can include a fruit ingredient as the food ingredient to make a viscous soluble fiber fortified food that is a fruit snack.

In aspect 10, some embodiments of aspect 9 make a viscous soluble fiber fortified food that is a gummy snack.

In aspect 11, some embodiments of any of aspects 6-10 include Ca2+ or Mg2+ in the divalent cation solution.

In aspect 12, a viscous soluble fiber fortified food is provided where the viscous soluble fiber fortified food has a moisture content of 10-30% and includes a viscous soluble fiber system, where the viscous soluble fiber system consists of:
a. a viscous soluble fiber source in an amount of at least 2% by dry weight of the food, the viscous soluble fiber source being partially hydrated;
b. an acid-reversible gel encapsulating the viscous soluble fiber source, the acid-reversible gel included in an amount providing a ratio of 30:1 to 6:1 by dry weight viscous soluble fiber source to acid-reversible gel, and maintaining the viscous soluble fiber source in a partially hydrated state over a shelf life of at least 30 days.

In aspect 13, some embodiments of aspect 12 include the viscous soluble fiber source in an amount of from about 5% to about 30% by weight of the food.

In aspect 14, some embodiments of aspect 12 or 13 psyllium husk is included in the viscous soluble fiber source comprises.

In aspect 15, some embodiments of any of aspects 12-14 include crosslinked pectin in the acid-reversible gel.

In aspect 16, some embodiments of any of aspects 12-15 include beta-glucan, guar gum, konjac, or hydroxypropyl methylcellulose in the viscous soluble fiber source.

In aspect 17, some embodiments of any of aspects 12-16 include crosslinked alginate in the acid-reversible gel.

In aspect 18, some embodiments of any of aspects 12-17 are a fruit snack.

In aspect 1, some embodiments of aspect 18 are a gummy fruit snack.

In aspect 20, some embodiments of 18 or 19 contain from about 5% to about 10% by weight viscous soluble fiber source.

In aspect 21, a method of making a texture-controlled fiber ingredient is provided where the method includes:
a. producing a homogenous composition consisting essentially of an aqueous plasticizer, a viscous soluble fiber source, and a non-crosslinked acid-reversible gel-forming compound, the viscous soluble fiber source and non-crosslinked acid-reversible gel-forming compound included at a ratio of 30:1 to 6:1 by dry weight viscous soluble fiber source to acid-reversible gel-forming compound; and
b. drying the composition to a moisture content of less than 12% to produce the texture-controlled fiber ingredient.

In aspect 22, some embodiments of aspect 21 perform part or all of step a at a temperature above 170° F.

In aspect 23, some embodiments of aspect 22 perform part or all of step a at a temperature from about 180° F to about 280° F.

In aspect 24, some embodiments of any of aspects 21-23 produce the homogenous composition having a moisture content of about 40% to about 90% by weight of the homogenous composition.

In aspect 25, some embodiments of any of aspects 21-24 dissolve the non-crosslinked acid-reversible gel-forming compound in the aqueous plasticizer to form a solution, and combine the solution with the viscous soluble fiber source.

In aspect 26, some embodiments of any of aspects 21-25 form the composition into pieces prior to drying.

In aspect 27, some embodiments of any of aspects 21-26 produce the homogenous composition in an extruder.

In aspect 28, some embodiments of aspect 27 produce the homogenous composition having a moisture content of about 50% to about 75% by weight of the homogenous composition.

In aspect 29, some embodiments of any of aspects 21-28 grind the texture-controlled fiber ingredient into a powder.

In aspect 30, some embodiments of any of aspects 21-24 dry the composition by spray drying.

In aspect 31, a fiber supplement is provided, including:
a. a viscous soluble fiber system, the viscous soluble fiber system consisting of a partially hydrated viscous soluble fiber source and an acid-reversible gel encapsulating the partially hydrated viscous soluble fiber source, the acid-reversible gel included at a ratio of 30:1 to 6:1 by dry weight viscous soluble fiber source to acid-reversible gel, and maintaining the viscous soluble fiber source in a partially hydrated state over a shelf life of at least 30 days;
b. a dehydrated viscous soluble fiber system, wherein the dehydrated viscous soluble fiber system comprises a viscous soluble fiber source and a non-crosslinked acid-reversible gel-forming compound at a ratio of 30:1 to 6:1 by dry weight viscous soluble fiber source to acid-reversible gel-forming compound; or
c. a texture-controlled fiber ingredient and a divalent cation, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound.

In aspect 32, some embodiments of aspect 31 include psyllium husk, beta-glucan, guar gum, konjac, or hydroxypropyl methylcellulose in the viscous soluble fiber source and include pectin or alginate in the acid-reversible gel-forming compound.

In aspect 33, some embodiments of aspect 31 include psyllium husk in the viscous soluble fiber source and pectin in the acid-reversible gel or acid-reversible gel-forming compound.

In aspect 34, some embodiments of any of aspects 31-33 have the fiber supplement with a moisture content of 10% or less.

In aspect 35, some embodiments of any of aspects 31-34 have the fiber supplement formulated to be hydrated prior to or during consumption.

In aspect 36, some embodiments of aspect 35 are a beverage mix, a chewable tablet, or a lozenge.

In aspect 37, some embodiments of any of aspects 31-33 have the fiber supplement with a moisture content of at least 10%.

In aspect 38, some embodiments of aspect 37 are a jelly or gummy supplement, a beverage, or a snack bar.

In aspect 39, some embodiments of any of aspects 31-38 include at least a portion of the texture-controlled fiber ingredient in a coating.

In aspect 40, some embodiments of any of aspects 31-39 include at least 1 g, or at least 1.7 g psyllium soluble fiber per serving.

In aspect 41, a method of relieving constipation in a patient is provided where the method comprises administering at least one serving of a fiber supplement according to aspect 40 at least once a day to a patient suffering from constipation until constipation is relieved.

In aspect 42, a method of reducing blood cholesterol levels in a patient is provided where the method comprises administering at least one serving of a fiber supplement according to aspect 40 at least once a day to a patient suffering high blood cholesterol levels until blood cholesterol levels in the patient are reduced.

In aspect 43, a method of increasing satiety in a patient is provided where the method comprising administering at least one serving of a fiber supplement according to aspect 40 to the patient prior to at least one meal a day, the fiber supplement containing an effective amount of psyllium husk to increase satiety.

In aspect 44, a fiber supplement for use in relieving constipation in a patient is provided where the fiber supplement includes at least 1 g, or at least 1.7 g psyllium soluble fiber per serving, and comprises:
a. a viscous soluble fiber system, the viscous soluble fiber system consisting of a partially hydrated psyllium husk and an acid-reversible gel encapsulating the partially hydrated psyllium husk, the acid-reversible gel included at a ratio of 30:1 to 6:1 by dry weight psyllium husk to acid-reversible gel, and maintaining the psyllium husk in a partially hydrated state over a shelf life of at least 30 days;
b. a dehydrated viscous soluble fiber system, wherein the dehydrated viscous soluble fiber system comprises psyllium husk and a non-crosslinked acid-reversible gel-forming compound at a ratio of 30:1 to 6:1 by dry weight psyllium husk to acid-reversible gel-forming compound; or
c. a texture-controlled fiber ingredient and a divalent cation, the texture-controlled fiber ingredient being an edible dry composition consisting essentially of psyllium husk and a non-crosslinked acid-reversible gel-forming compound at a ratio of 30:1 to 6:1 by dry weight viscous soluble fiber source to acid-reversible gel-forming compound.

In aspect 45, a fiber supplement for use in reducing blood cholesterol levels in a patient is provided where the fiber supplement includes at least 1 g, or at least 1.7 g psyllium soluble fiber, and comprises:
a. a viscous soluble fiber system, the viscous soluble fiber system consisting of a partially hydrated psyllium husk and an acid-reversible gel encapsulating the partially hydrated psyllium husk, the acid-reversible gel included at a ratio of 30:1 to 6:1 by dry weight psyllium husk to acid-reversible gel, and maintaining the psyllium husk in a partially hydrated state over a shelf life of at least 30 days;
b. a dehydrated viscous soluble fiber system, wherein the dehydrated viscous soluble fiber system comprises psyllium husk and a non-crosslinked acid-reversible gel-forming compound at a ratio of 30:1 to 6:1 by dry weight psyllium husk to acid-reversible gel-forming compound; or
c. a texture-controlled fiber ingredient and a divalent cation, the texture-controlled fiber ingredient being an edible dry composition consisting essentially of psyllium husk and a non-crosslinked acid-reversible gel-forming compound at a ratio of 30:1 to 6:1 by dry weight viscous soluble fiber source to acid-reversible gel-forming compound.

In aspect 46, a fiber supplement for use in increasing satiety in a patient is provided where the fiber supplement includes at least 6 g, or at least 7 g psyllium husk per serving, and comprises:
a. a viscous soluble fiber system, the viscous soluble fiber system consisting of a partially hydrated psyllium husk and an acid-reversible gel encapsulating the partially hydrated psyllium husk, the acid-reversible gel included at a ratio of 30:1 to 6:1 by dry weight psyllium husk to acid-reversible gel, and maintaining the psyllium husk in a partially hydrated state over a shelf life of at least 30 days;
b. a dehydrated viscous soluble fiber system, wherein the dehydrated viscous soluble fiber system comprises psyllium husk and a non-crosslinked acid-reversible gel-forming compound at a ratio of 30:1 to 6:1 by dry weight psyllium husk to acid-reversible gel-forming compound; or
c. a texture-controlled fiber ingredient and a divalent cation, the texture-controlled fiber ingredient being an edible dry composition consisting essentially of psyllium husk and a non-crosslinked acid-reversible gel-forming compound at a ratio of 30:1 to 6:1 by dry weight viscous soluble fiber source to acid-reversible gel-forming compound.

In aspect 47, some embodiments of any of aspects 31-46 include the viscous soluble fiber source in an amount of from about 2% to about 30% or about 3% to about 10% by dry weight of the fiber supplement.

In aspect 48, a method of making a fiber supplement according to any of aspects 31-47 is provided where the method includes:
a. combining an edible ingredient with a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound; contacting the texture-controlled fiber ingredient with a divalent cation solution in an amount sufficient to cross-link the acid-reversible gel-forming compound to form a first composition comprising the edible ingredient and a viscous soluble fiber system, the viscous soluble fiber system consisting of a partially hydrated viscous soluble fiber source and an acid-reversible gel encapsulating the partially hydrated viscous soluble fiber source; and forming the first composition into the fiber supplement;
b. combining the edible ingredient with a dehydrated viscous soluble fiber system to form a second composition, wherein the dehydrated viscous soluble fiber system comprises a viscous soluble fiber source and a non-crosslinked acid-reversible gel-forming compound at a ratio of 30:1 to 6:1 by dry weight viscous soluble fiber source to acid-reversible gel-forming compound; and forming the second composition into the fiber supplement; or
c. combining the edible ingredient with the texture-controlled fiber ingredient and a divalent cation source to form a third composition; and forming the third composition into the fiber supplement.

In aspect 49, some embodiments of aspect 48 combine the divalent cation solution with the edible ingredient prior to combining the edible ingredient with the texture-controlled fiber ingredient.

In aspect 50, some embodiments of aspect 48 contact the divalent cation solution with the texture-controlled fiber ingredient prior to combining the texture-controlled fiber ingredient with the edible ingredient.

In aspect 51, some embodiments of any of aspects 48-50 include Ca2+ or Mg2+ in the divalent cation solution.

In aspect 52, some embodiments of any of aspects 48-51 include a fruit ingredient in the edible ingredient to make a fruit-based fiber supplement.

In aspect 53, some embodiments of aspect 52 are a gummy fiber supplement.

In aspect 54, a method of making a dehydrated viscous soluble fiber system is provided, including:
a. providing a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound;
b. contacting the texture-controlled fiber ingredient with a divalent cation solution in an amount sufficient to cross-link the acid-reversible gel-forming compound, wherein contact of the divalent cation solution with the texture-controlled fiber ingredient is insufficient to cross-link at least a portion of the acid-reversible gel-forming compound; and
c. drying the viscous soluble fiber system to a moisture content of less than 12% to form the dehydrated viscous soluble fiber system, wherein the dehydrated viscous soluble fiber system includes the viscous soluble fiber source and at least a portion of the of the acid-reversible gel-forming compound that is non-crosslinked.

In aspect 55, a dehydrated viscous soluble fiber system is provided where the dehydrated viscous soluble fiber system is an edible dry composition consisting essentially of a viscous soluble fiber source, acid-reversible gel-forming compound, and a divalent cation in an amount sufficient to cross-link the acid-reversible gel-forming compound, the ratio of viscous soluble fiber source to acid-reversible gel-forming compound being 30:1 to 6:1 by dry weight, where at least a portion of the of the acid-reversible gel-forming compound is non-crosslinked.

In aspect 56, a method of making a viscous soluble fiber fortified food is provided where the method includes:
a. providing a food ingredient,
b. combining the food ingredient with a dehydrated viscous soluble fiber system, the dehydrated viscous soluble fiber system being an edible dry composition consisting essentially of a viscous soluble fiber source, acid-reversible gel-forming compound, and a divalent cation in an amount sufficient to cross-link the acid-reversible gel-forming compound, the ratio of viscous soluble fiber source to acid-reversible gel-forming compound being 30:1 to 6:1 by dry weight, where at least a portion of the of the acid-reversible gel-forming compound is non-crosslinked;
c. contacting the dehydrated viscous soluble fiber system with water in an amount sufficient to produce a viscous soluble fiber system in a composition, the viscous soluble fiber system consisting of:
   a. the viscous soluble fiber source in an amount of at least 2% by dry weight of the food, the viscous soluble fiber source being partially hydrated; and
   b. an acid-reversible gel encapsulating the viscous soluble fiber source, the acid-reversible gel maintaining the viscous soluble fiber source in a partially hydrated state over a shelf life of at least 30 days; and
d. producing a food from the composition.
In aspect 57, some embodiments of aspect 56 combine the water with the food ingredient prior to combining the food ingredient with the dehydrated viscous soluble fiber system.

In aspect 58, some embodiments of aspect 56 contact the water with the dehydrated viscous soluble fiber system prior to combining the dehydrated viscous soluble fiber system with the food ingredient.

In aspect 59, some embodiments of any of aspects 56-58 include the viscous soluble fiber source in an amount of from about 5% to about 30% by weight of the food.

In aspect 60, some embodiments of any of aspects 56-59 include psyllium husk in the viscous soluble fiber source.

In aspect 61, some embodiments of any of aspects 56-60 include crosslinked pectin in the acid-reversible gel.

In aspect 62, some embodiments of any of aspects 56-61 include beta-glucan, guar gum, konjac, or hydroxypropyl methylcellulose in the viscous soluble fiber source.

In aspect 63, some embodiments of any of aspects 56-62 include crosslinked alginate in the acid-reversible gel.

In aspect 64, a mix, kit, or product suitable for making a viscous soluble fiber fortified food is provided where the mix, kit, or product comprises at least one food ingredient, and:
a. a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound; or
b. dehydrated viscous soluble fiber system, the dehydrated viscous soluble fiber system being an edible dry composition consisting essentially of a viscous soluble fiber source, acid-reversible gel-forming compound, and a divalent cation in an amount sufficient to cross-link the acid-reversible gel-forming compound, the ratio of viscous soluble fiber source to acid-reversible gel-forming compound being 30:1 to 6:1 by dry weight, where at least a portion of the of the acid-reversible gel-forming compound is non-crosslinked.

In aspect 65, some embodiments of aspect 64 include the texture-controlled fiber ingredient and a divalent cation.

The following examples are provided to illustrate embodiments of the invention, and are not meant to be limiting.

### Examples

### Example 1 - Batch production of a texture-controlled psyllium ingredient

A 0.9% pectin solution was produced by combining low ester pectin powder (50/50 mix of pectin and sugar at 1.8% of the solution) with water, immersion blending the mixture at room temperature to disperse the pectin, and then heating the mixture to 180° F and immersion blending to form a solution. The solution was allowed to cool to a temperature less than 100° F (typically 70° F to less than 100° F). The solution was then added to the bowl of a mixer and psyllium husk was added quickly while mixing, followed by high speed mixing until well mixed to form a dough-like composition. The composition was then heated to boiling, the poured onto a silicone mat and formed into a sheet. The sheet was allowed to cool and solidify. The cooled sheet was cut into thin strips and dried at 145° F to a moisture of about 8%. The dried strips were ground into a powder to form a texture-controlled psyllium ingredient.

### Example 2 - Continuous production of a texture-controlled psyllium ingredient

A 4% pectin solution was produced by combining low ester pectin powder (50/50 mix of pectin and sugar at 8% by weight of the solution) with water and mixing at elevated temperature until the solution was formed. The pectin solution at ambient temperature was fed into a twin screw extruder to combine with ground psyllium husk to form a composition having a moisture content of about 50% and a ratio of 20:1 psyllium husk to pectin. Barrel temperatures ranged from 70° F to about 240° F. The composition was extruded and cut into pellets, dried to a moisture of 8-9% to form a texture-controlled psyllium ingredient.

In another test, texture-controlled psyllium ingredient was also successfully made in continuous production that included about 5% cocoa powder without affecting the functionality of the texture-controlled psyllium ingredient.

### Example 3 - Viscous soluble fiber fortified snacks using a texture-controlled psyllium ingredient

The texture-controlled psyllium ingredients made in Examples 1 and 2 were each used to produce a gummy snack and a fruit bite.

A gummy snack was made including fruit juice, gelatin, modified starches, syrup, sugar, sorbitol, flavor, color, water, calcium chloride, and texture-controlled psyllium ingredient. Briefly, all ingredients, except for gelatin, flavor, color and texture-controlled psyllium ingredient were cooked in a pressure cooker to 240° F for 40 minutes, then poured into pre-hydrated gelatin. The color and flavor were added and then the texture-controlled psyllium ingredient was added, and the ingredients were mixed to achieve a mixture containing about 6.7% psyllium husk by weight, about 0.16% calcium chloride by weight, and about 26% moisture. The hot mixture was deposited into starch molds and dried at 140° F for 10 hours, followed by another 48 hours at room temperature before removal. The final product had a moisture of about 14-15% and exhibited a soft, chewy eating texture with no viscous or slimy mouthfeel. The product had a pleasant taste and maintained the shape and definition of the starch mold.

A fruit bite was made with dried fruits (e.g., apples, pears, dates, prunes, etc.), nuts, nut butters, sorbitol, water, calcium chloride, and texture-controlled psyllium ingredient. Briefly, the calcium chloride was dissolved in water, followed by addition of sorbitol, which was then warmed to ensure sorbitol was dissolved. In some cases, the calcium chloride/sorbitol solution was stored at refrigerated temperature before combining with other ingredients. The texture-controlled psyllium ingredient was combined with the calcium chloride/sorbitol solution, followed by the remaining ingredients to form a dough-like composition containing approximately 6-7% psyllium husk and about 0.2% calcium chloride by weight. The composition was extruded through a hand-held jerky gun and cut into pieces to produce the fruit bites having a moisture content of about 21-22%. The fruit bites were observed to have a smooth surface with nuts and fruits visually apparent, and to exhibit a slightly chewy texture with no slimy or viscous mouthfeel over a room temperature shelf life of at least 30 days.

### Example 4 - Continuous production of a dehydrated viscous soluble fiber system

A texture-controlled psyllium ingredient was made as described in Example 2. Following extrusion, the pellets were dipped in a 2% calcium chloride solution for about 1 minute prior to drying to produce a dehydrated viscous soluble fiber system.

The dried pellets were tested to determine whether they would form a viscous soluble fiber system when combined with water. The pellets were ground into a powder and placed in an excess volume of water. The powder was observed to swell in the water, and swell further upon acidification of the water, indicating that a dehydrated viscous soluble fiber system could be successfully converted to a viscous soluble fiber system upon combining with water.

The dehydrated viscous soluble fiber system pellets were also added intact to a yogurt base with a pH 4.5 for 2 weeks to determine whether the dehydrated viscous soluble fiber system could produce a viscous soluble fiber system in which psyllium would remain partially hydrated over a period of time. The dehydrated viscous soluble fiber system pellets initially swelled to produce spheres that visually resembled tapioca pearls, then did not swell further for the test period. Upon eating, the tapioca pearl-like pellets had a texture similar to tapioca pearls and were not slimy or grainy. Without being bound by theory, it is believed that the dehydrated viscous soluble fiber system pellets were hydrated at the same time the pectin gel was crosslinked, resulting in a psyllium that was partially hydrated to form a viscous soluble fiber system. Although, the pH of the yogurt was low, it is believed that the pH was not low enough to result in the pectin failing to crosslink, or excess soluble calcium was available from the yogurt to maintain the pectin gel, or a combination of these factors, ensured that the resulting viscous soluble fiber system pearls included a partially hydrated psyllium over the test period.
Preferred Embodiments of the invention include the following:
Embodiment 1. A texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound.
Embodiment 2. The texture-controlled fiber ingredient of Embodiment 1, wherein the viscous soluble fiber source comprises psyllium husk, beta-glucan, guar gum, konjac, or hydroxypropyl methylcellulose, and the acid-reversible gel-forming compound comprises pectin or alginate.
Embodiment 3. The texture-controlled fiber ingredient of Embodiment 2, wherein the viscous soluble fiber source comprises psyllium husk and the acid-reversible gel-forming compound comprises pectin.
Embodiment 4. A method of making a viscous soluble fiber fortified food, the method comprising:
   a. providing a food ingredient,
   b. combining the food ingredient with a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound, and
   c. contacting the texture-controlled fiber ingredient with a divalent cation solution in an amount sufficient to cross-link the acid-reversible gel-forming compound.
Embodiment 5. The method of Embodiment 4, wherein the viscous soluble fiber fortified food is a gummy fruit snack.
Embodiment 6. A viscous soluble fiber fortified food, the viscous soluble fiber fortified food having a moisture content of 10-30% and including a viscous soluble fiber system, the viscous soluble fiber system consisting of:
   a. a viscous soluble fiber source in an amount of at least 2% by dry weight of the food, the viscous soluble fiber source being partially hydrated;
   b. an acid-reversible gel encapsulating the viscous soluble fiber source, the acid-reversible gel included in an amount providing a ratio of 30:1 to 6:1 by dry weight viscous soluble fiber source to acid-reversible gel, and maintaining the viscous soluble fiber source in a partially hydrated state over a shelf life of at least 30 days.
Embodiment 7. The food of Embodiment 6, wherein the viscous soluble fiber source is included in an amount of from about 5% to about 30% by weight of the food.
Embodiment 8. The food of Embodiment 6 or 7, wherein the viscous soluble fiber source comprises psyllium husk, beta-glucan, guar gum, konjac, or hydroxypropyl methylcellulose and the acid-reversible gel comprises crosslinked pectin or crosslinked alginate.
Embodiment 9. The food of Embodiment 8, wherein the viscous soluble fiber source comprises psyllium husk andthe acid-reversible gel comprises crosslinked pectin.
Embodiment 10. The food of any of Embodiments 6-9, wherein the food is a gummy fruit snack.
Embodiment 11. The food of Embodiment 10, wherein the fruit snack contains from about 5% to about 10% by weight viscous soluble fiber source.
Embodiment 12. A method of making a texture-controlled fiber ingredient, the method comprising:
   a. producing a homogenous composition consisting essentially of an aqueous plasticizer, a viscous soluble fiber source, and a non-crosslinked acid-reversible gel-forming compound, the viscous soluble fiber source and non-crosslinked acid-reversible gel-forming compound included at a ratio of 30:1 to 6:1 by dry weight viscous soluble fiber source to acid-reversible gel-forming compound; and
   b. drying the composition to a moisture content of less than 12% to produce the texture-controlled fiber ingredient.
Embodiment 13. The method of Embodiment 12, wherein the temperature is from about 180° F to about 280° F.
Embodiment 14. The method of Embodiment 12 or 13, wherein the homogenous composition has a moisture content of about 40% to about 90%.
Embodiment 15. The method of any of Embodiments 12-14, wherein the non-crosslinked acid-reversible gel-forming compound is dissolved in the aqueous plasticizer to form a solution, and the solution is combined with the viscous soluble fiber source.
Embodiment 16. A fiber supplement, comprising:
   a. a viscous soluble fiber system, the viscous soluble fiber system consisting of a partially hydrated viscous soluble fiber source and an acid-reversible gel encapsulating the partially hydrated viscous soluble fiber source, the acid-reversible gel included at a ratio of 30:1 to 6:1 by dry weight viscous soluble fiber source to acid-reversible gel, and maintaining the viscous soluble fiber source in a partially hydrated state over a shelf life of at least 30 days;
   b. a dehydrated viscous soluble fiber system, wherein the dehydrated viscous soluble fiber system comprises a viscous soluble fiber source and a non-crosslinked acid-reversible gel-forming compound at a ratio of 30:1 to 6:1 by dry weight viscous soluble fiber source to acid-reversible gel-forming compound; or
   c. a texture-controlled fiber ingredient and a divalent cation, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound.
Embodiment 17. The fiber supplement of Embodiment 16, wherein the viscous soluble fiber source comprises psyllium husk, beta-glucan, guar gum, konjac, or hydroxypropyl methylcellulose, and the acid-reversible gel-forming compound comprises pectin or alginate.
Embodiment 18. The fiber supplement of Embodiment 17, wherein the viscous soluble fiber source comprises psyllium husk and the acid-reversible gel or acid-reversible gel-forming compound comprises pectin.
Embodiment 19. The fiber supplement of any of Embodiments 16-18, wherein the fiber supplement has a moisture content of 10% or less.
Embodiment 20. The fiber supplement of Embodiment 19, wherein the supplement is a beverage mix, a chewable tablet, or a lozenge.
Embodiment 21. The fiber supplement of any of Embodiments 16-18, wherein the fiber supplement has a moisture content of at least 10%.
Embodiment 22. The fiber supplement of Embodiment 21, wherein the fiber supplement is a jelly or gummy supplement, a beverage, or a snack bar.
Embodiment 23. A fiber supplement for use in relieving constipation in a patient or for use in reducing blood cholesterol levels in a patient, the fiber supplement including at least 1 g, or at least 1.7 g psyllium soluble fiber per serving, and comprising:
   a. a viscous soluble fiber system, the viscous soluble fiber system consisting of a partially hydrated psyllium husk and an acid-reversible gel encapsulating the partially hydrated psyllium husk, the acid-reversible gel included at a ratio of 30:1 to 6:1 by dry weight psyllium husk to acid-reversible gel, and maintaining the psyllium husk in a partially hydrated state over a shelf life of at least 30 days;
   b. a dehydrated viscous soluble fiber system, wherein the dehydrated viscous soluble fiber system comprises psyllium husk and a non-crosslinked acid-reversible gel-forming compound at a ratio of 30:1 to 6:1 by dry weight psyllium husk to acid-reversible gel-forming compound; or
   c. a texture-controlled fiber ingredient and a divalent cation, the texture-controlled fiber ingredient being an edible dry composition consisting essentially of psyllium husk and a non-crosslinked acid-reversible gel-forming compound at a ratio of 30:1 to 6:1 by dry weight viscous soluble fiber source to acid-reversible gel-forming compound.
Embodiment 24. A fiber supplement for use in increasing satiety in a patient, the fiber supplement including at least 6 g, or at least 7 g psyllium husk per serving, and comprising:
   a. a viscous soluble fiber system, the viscous soluble fiber system consisting of a partially hydrated psyllium husk and an acid-reversible gel encapsulating the partially hydrated psyllium husk, the acid-reversible gel included at a ratio of 30:1 to 6:1 by dry weight psyllium husk to acid-reversible gel, and maintaining the psyllium husk in a partially hydrated state over a shelf life of at least 30 days;
   b. a dehydrated viscous soluble fiber system, wherein the dehydrated viscous soluble fiber system comprises psyllium husk and a non-crosslinked acid-reversible gel-forming compound at a ratio of 30:1 to 6:1 by dry weight psyllium husk to acid-reversible gel-forming compound; or
   c. a texture-controlled fiber ingredient and a divalent cation, the texture-controlled fiber ingredient being an edible dry composition consisting essentially of psyllium husk and a non-crosslinked acid-reversible gel-forming compound at a ratio of 30:1 to 6:1 by dry weight viscous soluble fiber source to acid-reversible gel-forming compound.
Embodiment 25. The fiber supplement of any of Embodiments 16-24, wherein the viscous soluble fiber source is included in an amount of from about 2% to about 30% or about 3% to about 10% by dry weight of the fiber supplement.
Embodiment 26. A method of making a fiber supplement of any of Embodiments 16-25, the method comprising:
   a. combining an edible ingredient with a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound; contacting the texture-controlled fiber ingredient with a divalent cation solution in an amount sufficient to cross-link the acid-reversible gel-forming compound to form a first composition comprising the edible ingredient and a viscous soluble fiber system, the viscous soluble fiber system consisting of a partially hydrated viscous soluble fiber source and an acid-reversible gel encapsulating the partially hydrated viscous soluble fiber source; and forming the first composition into the fiber supplement;
   b. combining the edible ingredient with a dehydrated viscous soluble fiber system to form a second composition, wherein the dehydrated viscous soluble fiber system comprises a viscous soluble fiber source and a non-crosslinked acid-reversible gel-forming compound at a ratio of 30:1 to 6:1 by dry weight viscous soluble fiber source to acid-reversible gel-forming compound; and forming the second composition into the fiber supplement; or
   c. combining the edible ingredient with the texture-controlled fiber ingredient and a divalent cation source to form a third composition; and forming the third composition into the fiber supplement.
Embodiment 27. A method of making a dehydrated viscous soluble fiber system, comprising:
   a. providing a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound;
   b. contacting the texture-controlled fiber ingredient with a divalent cation solution in an amount sufficient to cross-link the acid-reversible gel-forming compound, wherein contact of the divalent cation solution with the texture-controlled fiber ingredient is insufficient to cross-link at least a portion of the acid-reversible gel-forming compound; and
   c. drying the viscous soluble fiber system to a moisture content of less than 12% to form the dehydrated viscous soluble fiber system, wherein the dehydrated viscous soluble fiber system includes the viscous soluble fiber source and at least a portion of the of the acid-reversible gel-forming compound that is non-crosslinked.
Embodiment 28. A dehydrated viscous soluble fiber system, the dehydrated viscous soluble fiber system being an edible dry composition consisting essentially of a viscous soluble fiber source, acid-reversible gel-forming compound, and a divalent cation in an amount sufficient to cross-link the acid-reversible gel-forming compound, the ratio of viscous soluble fiber source to acid-reversible gel-forming compound being 30:1 to 6:1 by dry weight, where at least a portion of the of the acid-reversible gel-forming compound is non-crosslinked.
Embodiment 29. A method of making a viscous soluble fiber fortified food, the method comprising:
   a. providing a food ingredient,
   b. combining the food ingredient with a dehydrated viscous soluble fiber system, the dehydrated viscous soluble fiber system being an edible dry composition consisting essentially of a viscous soluble fiber source, acid-reversible gel-forming compound, and a divalent cation in an amount sufficient to cross-link the acid-reversible gel-forming compound, the ratio of viscous soluble fiber source to acid-reversible gel-forming compound being 30:1 to 6:1 by dry weight, where at least a portion of the of the acid-reversible gel-forming compound is non-crosslinked;
   c. contacting the dehydrated viscous soluble fiber system with water in an amount sufficient to produce a viscous soluble fiber system in a composition, the viscous soluble fiber system consisting of:
      a. the viscous soluble fiber source in an amount of at least 2% by dry weight of the food, the viscous soluble fiber source being partially hydrated; and
      b. an acid-reversible gel encapsulating the viscous soluble fiber source, the acid-reversible gel maintaining the viscous soluble fiber source in a partially hydrated state over a shelf life of at least 30 days; and
   d. producing a food from the composition.
Embodiment 30. The food of any of Embodiment 29, wherein the viscous soluble fiber source is included in an amount of from about 5% to about 30% by weight of the food.
Embodiment 31. The food of Embodiment 29 or 30, wherein the viscous soluble fiber source comprises psyllium husk and the acid-reversible gel comprises crosslinked pectin.
Embodiment 32. A mix, kit, or product suitable for making a viscous soluble fiber fortified food, the mix, kit, or product comprising at least one food ingredient, and:
   a. a texture-controlled fiber ingredient, the texture-controlled fiber ingredient being an edible dry composition consisting essentially of a viscous soluble fiber source and a non-crosslinked acid-reversible gel-forming compound at a ratio of 30:1 to 6:1 by dry weight viscous soluble fiber source to acid-reversible gel-forming compound; or
   b. dehydrated viscous soluble fiber system, the dehydrated viscous soluble fiber system being an edible dry composition consisting essentially of a viscous soluble fiber source, acid-reversible gel-forming compound, and a divalent cation in an amount sufficient to cross-link the acid-reversible gel-forming compound, the ratio of viscous soluble fiber source to acid-reversible gel-forming compound being 30:1 to 6:1 by dry weight, where at least a portion of the of the acid-reversible gel-forming compound is non-crosslinked.

## Claims

1. A fiber supplement, comprising:
a viscous soluble fiber system, the viscous soluble fiber system consisting of a partially hydrated viscous soluble fiber source and an acid-reversible gel encapsulating the partially hydrated viscous soluble fiber source, the acid-reversible gel included at a ratio of 30:1 to 6:1 by dry weight viscous soluble fiber source to acid-reversible gel, and maintaining the viscous soluble fiber source in a partially hydrated state over a shelf life of at least 30 days, wherein the viscous soluble fiber source is psyllium husk and the acid-reversible gel-forming compound is a pectin gel.

2. The fiber supplement of claim 1, wherein the fiber supplement has a moisture content of 10% or less.

3. The fiber supplement of claim 2, wherein the supplement is a beverage mix, a chewable tablet, or a lozenge.

4. The fiber supplement of claim 1, wherein the fiber supplement has a moisture content of at least 10%.

5. The fiber supplement of claim 4, wherein the fiber supplement is a jelly or gummy supplement, a beverage, or a snack bar.

6. The fiber supplement of claim 1, wherein the viscous soluble fiber source is included in an amount of from about 2% to about 30% by dry weight of the fiber supplement.

7. The fiber supplement of claim 1, wherein the viscous soluble fiber source is included in an amount of from about 3% to about 10% by dry weight of the fiber supplement

8. A method of making a fiber supplement claim 1, the method comprising:
combining an edible ingredient with a texture-controlled fiber ingredient, the texture-controlled fiber ingredient being an edible dry composition consisting essentially of the viscous soluble fiber source and a non-crosslinked acid-reversible gel-forming compound at a ratio of 30:1 to 6:1 by dry weight viscous soluble fiber source to acid-reversible gel-forming compound, wherein the acid-reversible gel-forming compound is pectin and the viscous soluble fiber source is psyllium husk;
contacting the texture-controlled fiber ingredient with a divalent cation solution in an amount sufficient to cross-link the acid-reversible gel-forming compound to form a composition comprising the edible ingredient and a viscous soluble fiber system, the viscous soluble fiber system consisting of the partially hydrated viscous soluble fiber source and the acid-reversible gel encapsulating the partially hydrated viscous soluble fiber source; and
forming the composition into the fiber supplement.
